# EUROPEAN PATENT APPLICATION

(11) **EP 3 542 700 A1**
(43) Date of publication of application: **25.09.2019**
(21) Application number: 18162524.5
(22) Date of filing: 19.03.2018
(51) Int. Cl.: A61B 1/005, A61B 1/008

(54) **ENDOSCOPE**

(71) Applicant: Adronic Endoscope Co., Ltd., 242 new Taipei City (TW)
(72) Inventor: TSENG, Hsiang-Te, 242 New Taipei City (TW)
(74) Representative: Viering, Jentschura & Partner mbB Patent- und Rechtsanwälte

(57) **Abstract**

An endoscope (100, 200) including a control device, an articulation joint (20), a detector (30) and at least one deconcentrator (40, 50) is disclosed. Wherein, the control device includes a control unit (12), a plurality of control strings (14) and a protective tube (16). The plurality of control strings (14) penetrates through the protective tube (16). One end of the plurality of control strings (14) is connected to the control unit (12), and another end of the plurality of the control strings (14) is connected to the articulation joint (20). The control unit (12) is controllable to pull the plurality of the control strings (14) and control the articulation joint (20) to bend. One end of the articulation joint (20) is connected to the protective tube (16), and another end of the articulation joint (20) is connected to the detector (30) such that the detector (30) would be moved together with the articulation joint (20) and the control strings (14) would not twine around each other.

## Description

### BACKGROUND OF THE INVENTION

### Technical Field

The present invention is related to an endoscope, and more particularly related to an endoscope which includes isolated control strings therein.

### Description of Related Art

An endoscope enables a user to detect an inside of a detection target without damaging the detection target. In recent years, the endoscope becomes popular and is widely utilized in every technical field to detect the inside of the detection target since the endoscope could detect the inside of the detection target without destroying it.

The conventional endoscope usually includes a flexible detection means to enable the user to detect the inside of the detection target since a non-flexible detection means easily results in a detection region of the inside of the detection target being hit or abraded by the detection means.

Wherein, the rotation of the detection means is achieved by a plurality of control strings driven via a control device of the endoscope which is controlled by the user.

However, when the user operates the control device to pull the control strings and drive the detection means to move, the control strings are easy to twine around each other due to multiple bending movements of the detection means.

It is probably that the user would not be able to control the detection means to bend to the desi red positi on when he operates the control device to pull the control strings and drive the detection means after the control strings twining around each other, or even worse, the detection means of the endoscope could not be controlled by the user due to the twining of the control strings.

### BRIEF SUMMARY OF THE INVENTION

In view of the above, an object of the present invention is to provide an endoscope which could prevent the control string from twining around each other so as to ensure that the endoscope coul d be operated normally.

The present invention provides an endoscope including a control device, an articulation joint, a detector and at least one deconcentrator. Wherein, the control device includes a control unit, a plurality of control strings and a protective tube. The plurality of control strings penetrates through the protective tube. One end of the plurality of control strings is connected to the control unit. The control unit is controllable to pull the plurality of the control strings. The articulation joint is connected to one end of the protective tube and is bendable, wherein another end of the plurality of the control strings is connected to the articulation joint. The detector is disposed at another end of the articulation joint which is corresponding to the protective tube. The at least one deconcentrator includes a plurality of holes, each of which is passed through by one of the plurality of control strings.

The advantage of the present invention is to prevent the control strings from twining around each other, which results in an incapability of controlling the articulation joint by the control unit.

### BRIEF DESCRIPTION OF THE SEVERAL VIEWS OF THE DRAWINGS

The present invention will be best understood by referring to the following detailed description of some illustrative embodiments in conjunction with the accompanying drawings, in which
FIG. 1 is a perspective view of an endoscope of a first embodiment according to the present i nventi on;
FIG. 2 is a sectional exploded view of the endoscope of the first embodi ment;
FIG. 3 is an exploded view of the endoscope of FIG. 1;
FIG. 4 is a cross-sectional view of the endoscope of FIG. 1 as viewed along line4-4 of FIG. 1;
FIG. 5 is a schematic view showing the plurality of control strings penetrating through the deconcentrator;
FIG. 6 is a sectional enlarged view of FIG. 5 showing the plurality of control strings passing through the deconcentrator;
FIG. 7 is a sectional enlarged view of FIG. 5 showing the plurality of control strings passing through the deconcentrator;
FIG. 8 is an exploded view of the articulation joint of the endoscope according to the first embodiment of the present invention;
FIG. 9 is a sectional enlarged view of FIG. 8; and
FIG. 10 is a schematic view showing that the deconcentrator is disposed in the protective tube of the endoscope according to a second embodiment of the present invention.

### DETAILED DESCRIPTION OF THE INVENTION

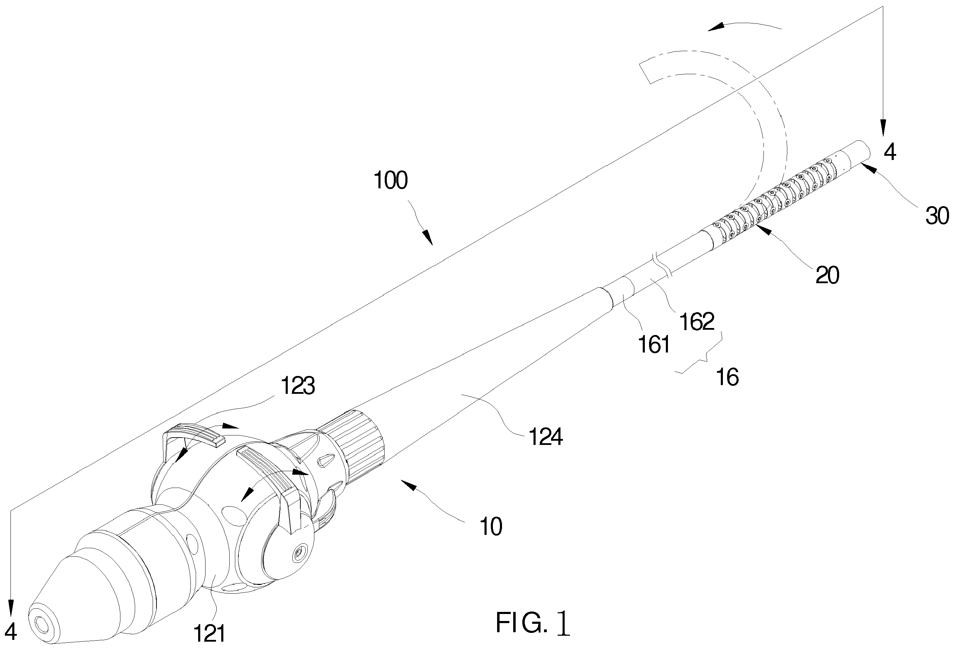

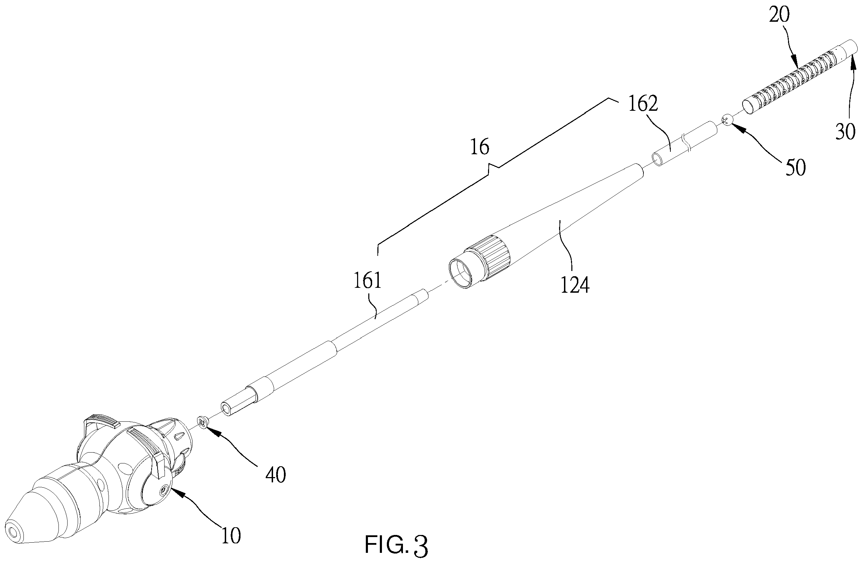

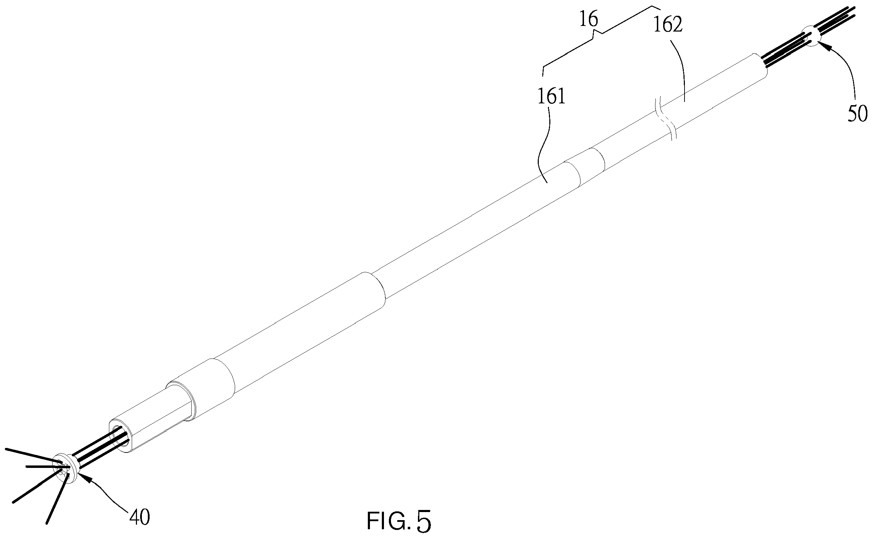

The following illustrative embodiments and drawings are provided to illustrate the disclosure of the present invention, these and other advantages and effects can be clearly understood by persons skilled in the art after reading the disclosure of this specification. FIG. 1 illustrates an endoscope 100 of a first embodi ment accordi ng to the present invention. The endoscope 100 is adapted to detect an inside of a detection target, wherein the detection target could be a living creature such as animals, or non-living thing such as a machine, however, this is not a limitation of the present invention. The endoscope 100 includes a control device 10, an articulation joint 20, a detector 30 and at least one deconcentrator (40, 50 as shown in FIG. 3 to FIG. 7), wherein the control device 10 is adapted to control the articulation joint 20 to rotate or bend, etc.; the detector 30 is disposed at a distal end of the articulation joint 20.

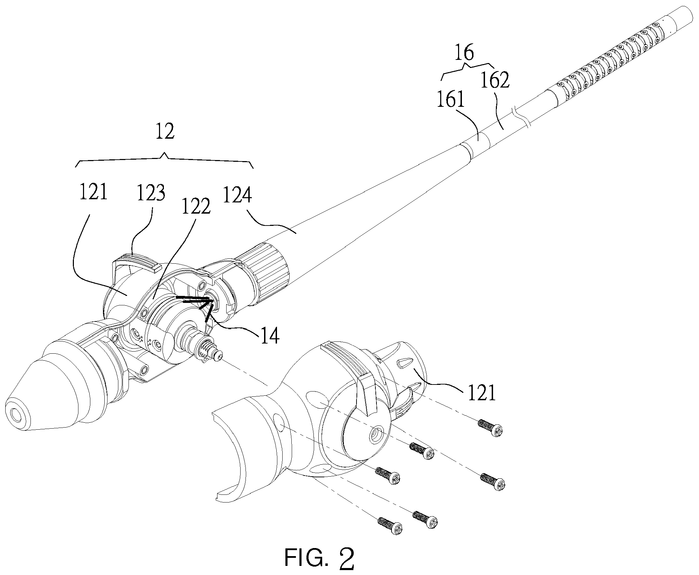

As illustrated in FIG. 2 and FIG. 3, the control device 10 includes a control unit 12, a plurality of control strings 14 and a protective tube 16. Wherein, the control strings 14 penetrate through the protective tube 16 such that the control strings 14 would not be damaged by an external friction force, or the inside of the detection target would not be damaged by the control strings 14. On end of the plurality of control strings 14 is connected to and controlled by the control unit 12, and the other end thereof is connected to the articulation joint 20. In the current embodiment, there are two pairs of control strings 14, wherein each pair includes two control strings 14 and is connected to a rotatable body 122 of the control unit 12, respectively. The rotatable body 122 is enclosed by two symmetrical half-housings 121 of the control unit 12. A control lever 123 is disposed on an exterior of each of the half-housings 121 respectively and could be pushed to rotate the rotatable body 122 so as to pull or release the control string 14, and thereby to change the direction of the articulation joint 20.

The protective tube 16 includes a first tube section 161 and a second tube section 162 which is flexible, wherein the first tube section 161 is disposed within an outer tube 124 of the control unit 1; one end of the first tube section 161 is proximal to the rotatable body 122 and the other end of the first tube section 161 is connected to the second tube section 162; the articulation joint 20 is connected to another end of the second tube section 162. Since the second tube section 162 is flexible, the second tube section 162 could be directed over a suitable deflection range when the control strings 14 are pulled to bend the articulation joint 20. In the current embodiment, when a user utilizes endoscope 100 to detect an inside of the detection target, the user could control the two control levers 123 to control the articulation joint 20 to adjust a proceeding direction and a detection direction of the endoscope 100 in the detection target.

Meanwhile, the second tube section 162 of protective tube 16 is not only adapted to protect the control strings 14 but also has an advantage of high flexibility when the endoscope 100 is used to detect the inside of the detection target. The detector 30 is disposed at the end of the articulation joint 20 which is connected to the protective tube 16, and therefore the detector 30 would be directed to another side of the flexible tube by the articulation joint 20 when the articulation joint 20 bends. As illustrated in FIG. 1, in the current embodiment, the detector 30 is immovably connected to the articulation joint 20.

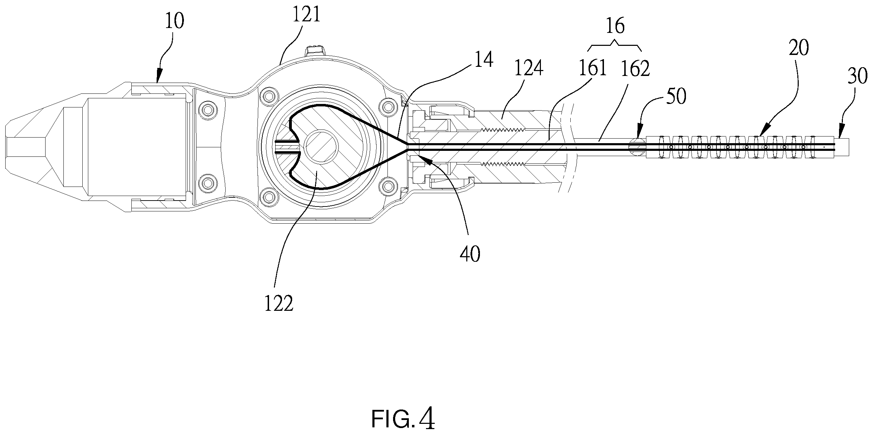

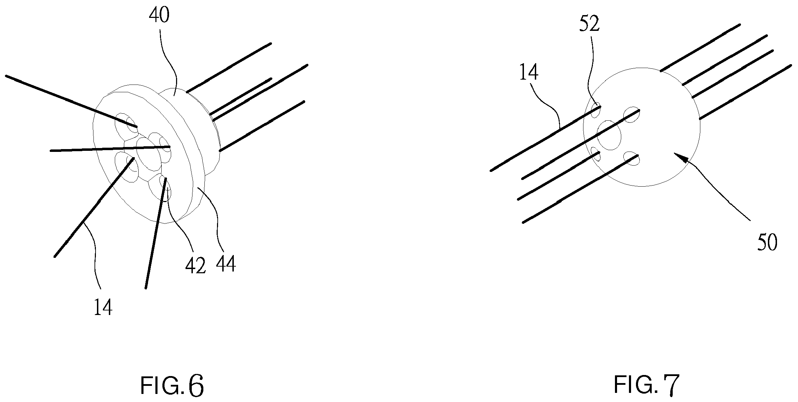

Referring to FIG. 4 and FIG. 7, there are two deconcentrators 40, 50 in the current embodiment, wherein the deconcentrator 40 is a disc shape and disposed at one end of the protective tube 16 which is proximal to the control unit 12; a rim flange 44 extends radially from the rim of the deconcentrator 40 and abuts against one end of the first tube 161 such that the deconcentrator 40 would not be fallen into the protective tube 16. The deconcentrator 50 is a ball shape and disposed at one end of the protective tube 16 which is proximal to the articulation joint 20. Each of the two deconcentrators 40, 50 includes a plurality of independent holes 42, 52 which respectively penetrate through the corresponding deconcentrator 40, 50. Each of the holes 42, 52 is adapted to be passed through by one of the control strings 14 such that the control strings 14 would not twine around each other. In other embodiments, the number of the deconcentrators could be increased depending on an increase of a length of the protective tube. In addition, the deconcentrators received in the protective tube could be all of the ball shape or disk shape, or could be a combination of disk shape and ball shape.

The deconcentrators 40, 50 are adapted to be passed through by the control strings 14 in order to prevent the control strings 14 f rom twining around each other, which results in an incapability of controlling the articulation joint 20 by the control unit 12. Wherein, to avoid the twining between the control strings 14, it is not limited to use two deconcentrators, and could use only one deconcentrator depending on the requirements. When a single one deconcentrator is bei ng utilized, the deconcentrator could be alternatively disposed at one end of the protective tube 16 which is proximal to the control unit 12 or the articulation joint 20, or disposed in the protective tube 16 between the control unit 12 and the articulation joint 20.

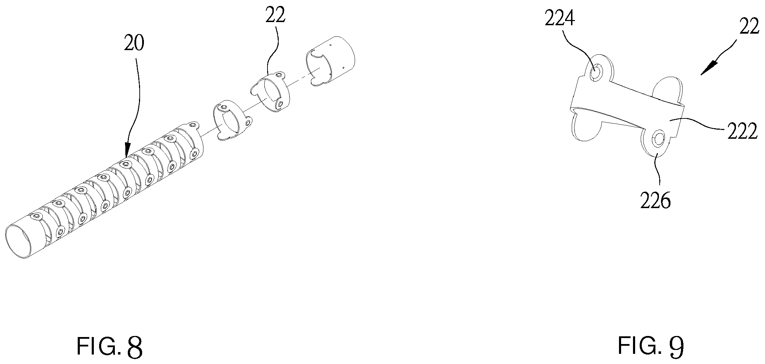

As illustrated in FIG. 8 and FIG. 9, in the current embodiment, the articulation joint 20 are formed by a plurality of link rings 22 which are engaged with each other. Each of the link ring 22 includes a ring body 222, two first tabs 224 extending from one side edge of the ring body 222, and two second tabs 226 extending from another side edge of the ring body 222. Wherein, the two first tabs 224 of one link ring 22 are pivotally connected to the two second tabs 226 of an adjacent link ring 22; a gap is formed between every two neighboring link rings 22 such that a moving space of the link rings 22 could be increased, thereby improving the flexibility of the articulation joint 20 which enables the articulation joint 20 to be controlled to bend in arbitrary angles.

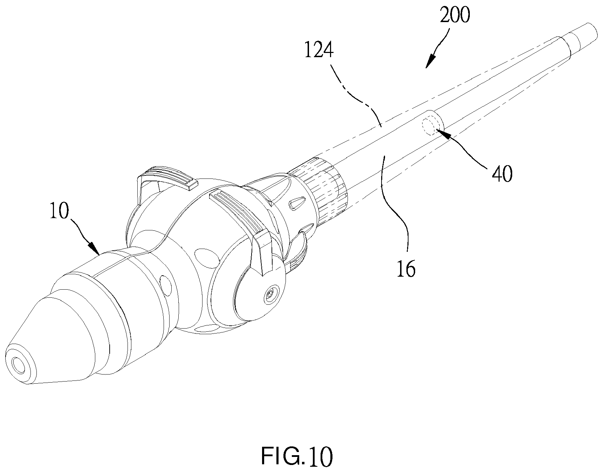

FIG. 10 illustrates an endoscope 200 of a second embodi ment accordi ng to the present invention. The endoscope 200 of the second embodiment includes only one deconcentrator 40, and the deconcentrator 40 is disposed in the protective tube 16. Even though the deconcentrator 40 of the second embodiment is a disk shape as an example, however, the deconcentrator 40 also could be a ball shape.

It must be pointed out that the embodiments described above are only some embodiments of the present invention. All equivalent structures which employ the concepts disclosed in this specification and the appended claims should fall within the scope of the present invention.

## Claims

1. An endoscope (100, 200), comprising:
a control device (10) including a control unit (12), a plurality of control strings (14) and a protective tube (16), wherein the plurality of control strings (14) penetrates through the protective tube (16); one end of the plurality of control strings (14) is connected to the control unit (12); the control unit (12) is controllable to pull the plurality of the control strings (14);
an articulation joint (20), being connected to one end of the protective tube (16) and bendable, wherein another end of the plurality of the control strings (14) is connected to the articulation joint (20);
a detector (30) disposed at another end of the articulation joint (20) which is correspondi ng to the protective tube (16); and
at least one deconcentrator (40, 50) including a plurality of holes (42, 52), each of which is passed through by one of the plurality of control strings (14).

2. The endoscope (100, 200) of claim 1, wherein the deconcentrator (40, 50) is disposed at one end of the protective tube (16) which is proximal to the control unit (12).

3. The endoscope (100, 200) of claim 1, wherein the deconcentrator (40, 50) is disposed at one end of the protective tube (16) which is proximal to the articulation joint (20).

4. The endoscope (100, 200) of claim 1, wherein the deconcentrator (40, 50) is disposed in the protective tube (16) between the control unit (12) and the articulation joint (20).

5. The endoscope (100, 200) of claim 1, wherein the deconcentrator (40) is a disk shape.

6. The endoscope (100, 200) of claim 1, wherein the deconcentrator (40) is a disk shape and disposed at one end of the protective tube (16) which is proximal to the control unit (12).

7. The endoscope (100, 200) of claim 6, wherein the deconcentrator (40, 50) includes a rim flange (44) which extends radially and abuts against the end of the protective tube (16).

8. The endoscope (100, 200) of claim 1, wherein the deconcentrator (50) is a ball shape.

9. The endoscope (100, 200) of claim 1, wherein the deconcentrator (50) is a ball shape and disposed at one end of the protective tube (16) which is proximal to the articulation joint (20).

10. The endoscope (100, 200) of claim 1, wherein the endoscope (100, 200) comprises two deconcentrators (40, 50), wherein one of the two deconcentrators (40, 50) is disposed at one end of the protective tube (16) which is proximal to the control unit (12), and the other one is disposed at one end of the protective tube (16) which is proximal to the articulation joint (20).

11. The endoscope (100, 200) of claim 1, wherein the articulation joint (20) comprises a plurality of link rings (22) which are engaged with each other and a gap is formed between every two neighboring link rings (22).

12. The endoscope (100, 200) of claim 11, wherein each of the plurality of link rings (22) comprises a ring body (222), two first tabs (224) extending from one side edge of the ring body (222), and two second tabs (226) extending from another side edge of the ring body; two first tabs (224) of one of the plurality of link rings (22) is pivotally connected to two second tabs (226) of another neighboring one of the plurality of link rings (22).

13. The endoscope (100, 200) of claim 1, wherein the control unit (12) comprises two control levers (123) and two rotatable bodies (122); each of the control levers (123) is adapted to control one of the rotatable bodies (122) to rotate and is connected to at least two of the plurality of the control strings (14).
